# EUROPEAN PATENT APPLICATION

(11) **EP 2 468 706 A1**
(43) Date of publication of application: **27.06.2012**
(21) Application number: 10195211.7
(22) Date of filing: 15.12.2010
(51) Int. Cl.: C07C 37/055, C07C 39/215, C07C 67/313

(54) **Process for the preparation of resveratrol**

(71) Applicant: DSM IP Assets B.V., 6411 TE Heerlen (NL)
(72) Inventor: Wehrli, Christof, CH-4002 Basel (CH)
(74) Representative: Kurt, Manfred

(57) **Abstract**

Process for the preparation of resveratrol by reacting a solution of acetylated 1-(3.5-dihydroxyphenyl)-bromoethane in DMA or NMP containing alkaline or alkaline earth carbonate or phosphate with a solution of acetylated p-bromophenol in the same solvent containing alkaline or alkaline earth carbonate or phosphate and a catalytic amount of palladium oximate under heating, addition of water and optionally isolating the resveratrol and purifying it.

## Description

The present invention is concerned with a novel process for the preparation of resveratrol.

Resveratrol, by systematic name 3.4'.5-trihydroxystilbene, is a well-known compound occurring in nature which has gained high interest because of its valuable biological properties, particularly of the (E)-isomer.

Processes for the preparation of resveratrol, particularly of the (E)-isomer, have been disclosed, inter alia, in WO 01/60774 and Tetrahedron Letters 43 (2002), 597-598. The latter reference describes a process wherein resveratrol is prepared by a multistep reaction sequence starting from 3.4'.5-dihydroxybenzaldehyde and involving a Heck reaction of 3,5'-diacetoxystyrene with 4-acetoxy-iodobenzene in a yield of 70 %.

The process for the preparation of resveratrol, as described, e.g., in US 7,820,848 utilizes a more readily available starting material and provides a technically more attractive approach in the preparation of resveratrol in higher yield and less process steps. This approach starts from 3.5-diacetylacetophenone (DAK) which is accessible from 3,5-dihydroxybenzoic acid. Formation of 1-(3.5-diacetoxyphenyl)-ethanol (DAL) by hydrogenation of DAK with Raney nickel proceeds in high yield. Bromination of DAL with 0.35 equivalents of phosphorous tribromide in toluene forms the bromide 1-(3.5-diacetoxyphenyl)-bromoethane (BrDA). Dehydrobromination of the resulting BrDA was achieved with a mixture of lithium bromide and lithium carbonate forming 3.5-diacetoxystyrene (DAS). The reaction mixture was poured into ethylacetate, the DAS was extracted and purified to a colorless oil. Reacting DAS with p-bromophenol-acetate (PBA) under conditions of a Heck reaction, in the presence of sodium bicarbonate, tri-o-tolylphosphine and Pd-acetate and acetylation of the raw product after extraction with ethylacetate yielded (E)-3.5.4'-triacetylresveratrol. Hydrolysis of the acetate by a huge excess of a methanolic solution of ammonium acetate and isolation of the product obtained finalises the synthesis of resveratrol. For the person skilled in the art, this process still comprises some drawbacks, e.g., three separate reaction steps starting from BrDA with the use of different solvents, two extractions with ethylacetate and high amount of ammonium acetate needed. The present invention, therefore, results from an effort to eliminate such drawbacks and at the same time improve the overall yield of resveratrol.

ln accordance with the present invention a surprising improvement has been found in that three process steps are combined into one step and can be performed as a one-pot reaction or throughput process.

More precisely the present invention relates to a process for the preparation of resveratrol, characterized by treating a solution of completely or partly acetylated 1-(3.5-dihydroxyphenyl)-bromoethane (BrDA) in dimethylacetamide (DMA) or N-methylpyrrolidone (NMP) first with an alkaline or alkaline earth carbonate or phosphate, preferably at about 80 °C to 170 °C, followed by reacting the such treated completely or partly acetylated 1-(3.5-diacetoxyphenyl)-bromoethane with a solution of partly or completely acetylated p-bromophenol (PBP) in the same solvent in the presence of an alkaline carbonate or phosphate and catalytic amounts of palladium oximate, under heating to 80 °C to 150 °C (preferred 100 °C to 170 °C), followed by addition of water in order to hydrolyze the acetoxy groups of the reaction product and, optionally, by isolating the resveratrol obtained from the reaction mixture and, further optionally, by purifying it.

Surprisingly it was found, that by the combination of specific amounts of alkaline or alkaline earth carbonate or phosphate (perferred 0.5 mole of lithiumcarbonate) used in the first step (the dehydrobromination) with an excess of potassium phosphate or potassium carbonate used in the second step (the Heck reaction) higher yields of resveratrol were obtained if all steps, including hydrolysis, are combined to a single throuput reaction, in comparison with a reaction sequence wherein resveratrol is produced in three isolated steps and wherein other combinations of alkaline compounds are used, e.g., calciumcarbonate in the first step and potassium carbonate in the second step.

The present invention comprises the mixing of two solutions each containing a partly or completely acetylated compound. This means in case of p-bromophenol (PBP) that the solution contains only p-bromophenol-acetate (PBA) or a mixture of p-bromophenol and p-bromophenol-acetate. In case of 1-(3.5-dihydroxyphenyl)-bromoethane this means that the solution contains only 1-(3.5-diacetoxyphenyl)-bromoethane (BrDA) or only 1-(3-acetoxy-5-hydroxy-phenyl)-bromoethane or mixtures of 1-(3.5-diacetoxyphenyl)-bromoethane with 1-(3-acetoxy-5-hydroxy)-bromoethane and/or 1-(3.5-dihydroxy)-bromoethane. Best results are obtained using completely acetylated reactants.

In principle inert organic solvents such as hydrocarbons, ethers, nitriles, esters, ketones or amides can be used in the present reaction sequence, however, it has been found that amides, particularly N,N-dimethylacetamide (DMA) and N-methylpyrrolidone (NMP) are preferred and give best results, DMA being the most preferred solvent in view of higher yields and easier recovery.

The reaction mixtures contain suitably at least one inorganic or organic base. Examples of inorganic bases are alkaline or alkaline earth carbonates, hydrogencarbonates and phosphates, preferably Li, Na and K carbonates or phosphates. Examples of organic bases are amines, such as N-methylmorpholine, triethyl-amine or diisopropyl-ethylamine, or alkali acetates, such as sodium acetate. Preferred base are lithium carbonate, potassium carbonate and potassium phosphate. The bases are used in at least about half equimolar to about two fold molar excess based on the reactands.

The first reaction step of the present throughput process, the dehydrobromination of BrDA, proceeds best with about the theoretical amount of lithium carbonate (0.45 to 0.6 mole equivalents).

The second reaction step of the present throughput process, the Heck reaction, proceeds with a second base (0.5 to 4 mole equivalents), preferred with an excess of base of 1 to 4 mole equivalents of potassium carbonate or potassium phosphate. In a preferred embodiment about 2 equivalents of base are used, so that the surplus of base is sufficient to hydrolyse the acetate groups after the addition of water in the last step of the throughput reaction. The reaction of the reactants is effected at a temperature in the range of 100 °C to 150 °C, preferably at about 130 °C, until nearly quantitative reaction (> 95 %). Before hydrolysis of the reaction product by addition of water it is recommendable to distill off the main part of the solvent under reduced pressure which is then recycled.

According to general conditions of a Heck reaction a catalyst comprising a Pd source, such as palladium acetate or Pd(dba)₂ and a stabilizing ligand or oxime-derived Pd complexes are used. In connection with the present invention palladium oximate (CAS 32679-19-9) has proven to be the best choice. The amount of the catalyst is in the range of 0.01 to 1 mole-%, preferably 0.05 to 0.2 mole-% equivalents of Pd.

The use of about equimolar amounts of the reactants is advantageous.

The third step of the present throughput reaction, the hydrolysis of the acetates is effected by addition of water, optionally by addition of a base (like an alkaline hydroxide or carbonate, e.g., sodium or potassium hydroxide or carbonate).

From the reaction mixture resveratrol can be isolated using methods well-known in the art, e.g., by evaporation of the solvent and extraction with a suitable solvent, e.g., ethylacetate. Furthermore, if desired, the resveratrol thus obtained can be further purified to high purity by methods well-known in the art.

The invention is described in more detail by the following Examples.

### Example 1

ln a reaction flask with stirrer were provided under nitrogen 16.0 mmole of 1-(3.5-diacetoxyphenyl)-bromoethane, 0.606 g of lithium carbonate (Fluka; 8.2 mmole) and 22 ml of DMA (Fluka). The mixture was heated at 120 °C under stirring for 2 hours. The clear solution was cooled to ambient temperature.

In a reaction flask with stirrer were provided under nitrogen 4.86 g of potassium carbonate (Fluka; 35.2 mmole), 3.81 g of PBA (96 w-%; 17.0 mmole), 10 ml DMA and 4.4 mg of Pd-oximate (16.0 µmole Pd). The reaction mixture was stirred at 130 °C, the above DMA solution was added in 45 minutes while heating at 130 °C and stirring for additional 2.5 hours. The main part of the solvent (24.0 g) was distilled at about 20 mbar until the residual mixture got viscous. 3.4 ml of water were added to the brownish residue and the mixture was stirred for about 2 hours at 115 °C until complete hydrolysis of the acetates. This mixture was diluted at 50 °C with 25 g of water and 30 g of ethylacetate. The mixture was stirred at ambient temperature for 30 minutes and filtered. The lower phase of the filtrate was separated and extracted with 45 g of ethylacetate. The two organic phases were washed in series with 4 x 25 ml of brine. The organic phases were combined and evaporated under vacuum. The residue of 4.28 g contained 65.1 % of resveratrol (HPLC; yield: 76 mole-%).

### Example 2

To a reaction flask with stirrer were provided under nitrogen 16.0 mmole of 1-(3.5-diacetoxyphenyl)-bromoethane, 0.606 g of lithium carbonate (Fluka; 8.2 mmole) and 18 ml of NMP (Fluka). The mixture was heated at 120 °C under stirring for 2 hours. The clear solution was cooled to ambient temperature.

In a reaction flask with stirrer were provided under nitrogen 4.68 g of potassium carbonate (Fluka; 35.2 mmole), 3.81 g of PBA (96 w-%; 17.0 mmole), 10 ml of NMP and 4.4 mg of Pd-oximate (16.0 µmole Pd). The reaction mixture was stirred at 130 °C. Then the above NMP solution was added in 45 minutes while heating at 130 °C and stirring for additional 1.5 hours. After removal of the main part of the solvent by distillation 3.4 ml of water were added and the mixture was stirred for 2 hours at 115 °C to complete hydrolysis of the acetates. To this mixture were added 50 g of water and 40 g of ethylacetate. The mixture was stirred for 10 minutes and the lower phase was separated and extracted with 50 g of ethylacetate. The two organic phases (in round bottom flasks with magnetic strirrers) were washed with 4 x 25 ml of brine. The organic phases were combined and evaporated under vacuum. The residue of 4.16 g contained 58.8 % of resveratrol (HPLC; yield: 67 mole-%).

### Example 3

A reaction flask with stirrer was provided under nitrogen with 16.0 mmole of 1-(3.5-diacetoxyphenyl)-bromoethane, 0,606 g of lithium carbonate (Fluka; 8.2 mmole) and 16 ml of DMA. The mixture was heated at 120 °C under strirring for 2 hours. The clear solution was cooled to ambient temperature.

ln a reaction flask with stirrer were provided under nitrogen 7.47 g of potassium phosphate (Riedel-de Haen; tribasic, water-free, 35.2 mmole), 3.81 of PBA (96 w-%; 17.0 mmole), 21 ml of DMA and 4.4 mg of Pd-oximate (16.0 µmole Pd). The reaction mixture was inertised and stirred at 130 °C. Then the above DMA solution was added in 45 minutes under heating at 130 °C and stirring for additional 19 hours at 130 °C, until > 98 % turnover of the intermediate styrene. The main part of the solvent was distilled at about 20 mbar until the residual mixture in the reactor got viscous. 13 ml of water and 2.2 g of potassium carbonate were added to the warm, brownish residue and the mixture was stirred for about 2.5 hours at 110 °C until complete hydrolysis of the acetates. To the viscous mixture were added at 50 °C 20 g of water and 30 g of ethylacetate. The slurry was filtered and washed with ethylacetate. The two phases were separated. The lower phase was extracted with 45 g of ethylacetate. The two organic phases were washed with 4 x 25 ml of brine. The organic phases were combined and evaporated at reduced pressure. The residue of 4.02 g contained 70.5 % of resveratrol (yield: 77 mole-%). Upon treatment with ethanol/water 2.78 g of resveratrol (content 99 %) were obtained.

## Claims

1. Process for the preparation of resveratrol, **characterized by** reacting a solution of completely or partly acetylated 1-(3.5-dihydroxyphenyl)-bromoethane in dimethylacetamide or N-methylpyrrolidone first with an alkaline or alkaline earth carbonate or phosphate followed by reacting the such treated completely or partly acetylated 1-(3.5-dihydroxyphenyl)-bromo-ethane with a solution of completely or partly acetylated p-bromophenol in the same solvent in the presence of an alkaline or alkaline earth carbonate or phosphate and catalytic amounts of palladium oximate under heating to 80°C to 170°C, followed by addition of water in order to hydrolyze the acetoxy groups of the reaction product and, optionally, by isolating the resveratrol obtained from the reaction mixture and, further optionally, by purifying it.

2. The process of claim 1 wherein (E)-resveratrol is obtained.

3. The process of claim 1 or claim 2 wherein 1-(3.5-diacetoxyoxyphenyl)-bromoethane and p-bromophenol-acetate are reacted.

4. The process of any of the preceding claims wherein dimethylacetamide or N-methylpyrrolidone is used as solvent.

5. The process of any of the preceding claims wherein the solution of acetylated 1-(3.5-dihydroxyphenyl)-bromoethane contains lithium carbonate and the solution of acetylated p-bromophenol contains potassium phosphate or carbonate.

6. The process of claim 5 wherein the solution of acetylated 1-(3.5-dihydroxyphenyl)-bromoethane containing lithium carbonate is added to the solution of acetylated p-bromophenol containing potassium phosphate or carbonate

7. The process of any of the preceding claims wherein the solution of the acetylated p-bromophenol contains the carbonate or phosphate base in excess sufficient to hydrolyse the acetoxy groups quantitatively.

8. The process of any of the preceding claims wherein the hydrolysis of the acetate groups proceeds by addition of water to the reaction mixture, optionally after the removal of the solvent

9. The process of any of the preceding claims wherein resveratrol is isolated from the reaction mixture and purified.

10. The process of claim 9 wherein isolation of resveratrol from the reaction mixture is achieved by evaporation of the solvent and extraction with ethylacetate.
